# EUROPÄISCHE PATENTSCHRIFT

(11) **EP 2 090 273 B1**
(45) Veröffentlichungstag und Bekanntmachung des Hinweises auf die Patenterteilung: **12.03.2014**
(21) Anmeldenummer: 09001945.6
(22) Anmeldetag: 12.02.2009
(51) Int. Cl.: A61F 13/10, A61F 13/06

(54) **Bandage**
Bandage
Bandage

(30) Priorität: 15.02.2008 DE 102008009635
(43) Veröffentlichungstag der Anmeldung: 19.08.2009
(73) Patentinhaber: Otto Bock HealthCare GmbH, 37115 Duderstadt (DE)
(72) Erfinder: Kroll-Orywahl, Olaf, 37085 Göttingen (DE); Reinhardt, Holger, 47906 Kempen (DE); Schlomski, Jens, 37115 Duderstadt (DE)
(74) Vertreter: Stornebel, Kai

(56) Entgegenhaltungen:
- EP-A1- 0 775 476
- WO-A1-86/04811
- WO-A1-99/00076
- US-A- 4 700 698

## Beschreibung

Die Erfindung betrifft eine Bandage, insbesondere eine Handgelenkbandage, mit einem elastischen Schlauch aus Gestrick, der ausgebildet ist, um an einem Körpergelenk anzuliegen.

Eine derartige Handgelenkbandage ist aus der EP 0 369 397 B1 bekannt und dient dazu, das Handgelenk, beispielsweise bei einem Karpaltunnel-Syndrom, ruhigzustellen. Nachteilig an der bekannten Handgelenkbandage ist, dass sie schlecht anzuziehen ist. Gerade dann, wenn das Handgelenk bei Bewegung schmerzt, ist es besonders wichtig, dass beim Überstreifen der Handgelenkbandage nur möglichst kleine Kräfte entstehen.

Aus der DE 103 58 146 A1 ist eine elastische Bandage bekannt, die einen elastischen Schlauch umfasst. Der Schlauch hat eine Vorder- und eine Beugeseite, wobei die Beugeseite zumindest bereichsweise zweilagig ausgebildet ist. Dadurch schnürt die Bandage das Körpergelenk, an dem sie anliegt, besonders wenig ein. Nachteilig hieran ist, dass die Bandage an Knochenvorsprüngen eine unangenehm hohe Kompression ausüben kann.

Aus der DE 42 37 389 A1 ist eine Kniegelenkbandage bekannt, bei der eine Pelotte eingesetzt wird, um die Kompressionswirkung der Kniegelenkbandage auf die Patella zu verringern. Nachteilig hieran ist, dass es im Bereich der Pelotte zu einem Drucksprung kommt. Das heißt, dass die Kompressionswirkung der Kniegelenkbandage mit abnehmendem Abstand zur Patella zunächst im Bereich der Pelotte zunimmt und dann schlagartig abnimmt. Das mindert den Tragekomfort und ist daher unerwünscht.

Die EP 0 775 476 A1 betrifft eine Handgelenksbandage aus einem flachen Zuschnitt mit Klettverschlüssen, um um das Handgelenk herum angelegt und befestigt zu werden. Taschen für feste Schienen sind an der Außenseite eingearbeitet. Zum Stabilisieren des Handgelenkes wird das flächige Material um das Handgelenk gelegt und über Klettverschlüsse fixiert.

Die WO 99/00076 A1 betrifft eine Karpaltunnerorthese mit einem elastischen Band, auf dessen Innenseite Kompressionselemente aufgeklettet sind.

Die WO 86/04811 A1 betrifft eine Epicondylitisbandage mit einem Schlauchabschnitt, auf dessen Außenseite plattenförmige Einlagen mittels eines Spannriehmens, der in Umfangsrichtung verläuft, gegen den Unterarm gedrückt werden.

Die US 4,700,698 A betrifft eine Knieorthese mit einer Ringpelotte um den Patellabereich. In der Ringpelotte sind Verstärkungselemente eingearbeitet.

Der Erfindung liegt die Aufgabe zugrunde, eine Bandage vorzuschlagen, die besonders einfach anzuziehen ist und leicht an den Gesundungsfortschritt angepasst werden kann.

Die Erfindung löst das Problem durch eine gattungsgemäße Bandage, bei der der Schlauch an einer Knochenvorsprungstelle, die bei Benutzung an einem Knochenvorsprung anliegt, eine geringere Kompressionswirkung aufweist als jenseits der Knochenvorsprungstelle, und dass die Bandage ein Stabilisierungselement, das zum Fixieren an der Knochenvorsprungstelle ausgebildet ist, umfasst. Das Stabilisierungselement überbrückt dabei im angelegten Zustand das Körpergelenk zumindest teilweise, um die Bewegung des Körpergelenks in zumindest einer Richtung zu hemmen. Das Stabilisierungselement, das als formstabiles, in zumindest einer Richtung einen Verformungswiderstand ausbildendes Element ausgestattet ist, dient dazu, dass das Gelenk in seiner Beweglichkeit eingeschränkt wird. Dadurch kann bei einer Verletzung oder Überbeanspruchung das Gelenk ruhiggestellt oder zumindest in seinem Bewegungsumfang eingeschränkt werden, sofern dies aus medizinischer Sicht notwendig ist. Eine besonders angenehm zu tragende Bandage wird erhalten, wenn das Stabilisierungselement bzw. die Pelotte eine Ausnehmung aufweist, die in ihren Abmessungen im Wesentlichen dem Knochenvorsprung entspricht. Vorteilhafterweise kann diese Ausnehmung dazu verwendet werden, um das Stabilisierungselement in ihrer korrekten Lage relativ zum Knochenvorsprung zu positionieren. Durch die Ausnehmung wird das Stabilisierungselement zudem beim Tragen in der Bandage am Ort gehalten.

Vorteilhaft an der erfindungsgemäßen Bandage ist, dass durch das Stabilisierungselement die Notwendigkeit entfällt, die Stabilisierungswirkung über den elastischen Schlauch auszuüben, was Pelotten oder Öffnungen zur Druckentlastung notwendig macht. Da keine Pelotten oder Öffnungen, die auch als Ödemfenster bezeichnet notwendig sind, werden Drucksprünge vermieden.

Wenn die Bandage eine Handgelenkbandage ist, so kann diese Handbandage dann besonders angenehm getragen werden, wenn kein übermäßig starker Druck an der Knochenvorsprungstelle, die in diesem Fall die Griffelfortsatzstelle ist, anliegt. Bei bekannten Bandagen wird die Umgebung der Knochenvorsprungstelle gepolstert. Dadurch konzentriert sich der von der Bandage ausgeübte Druck auf die Umgebung der Knochenvorsprungstelle und die Knochenvorsprungstelle selbst wird entlastet. Diese Polsterung stört jedoch beim Anziehen, was die erfindungsgemäße Bandage vermeidet.

Das Stabilisierungselement ist zur leichteren Anpassung an den Gesundungsgrad des Bandagenträgers abnehmbar an der Oberfläche des Schlauches befestigt, beispielsweise auf der Außenseite des Schlauches. Grundsätzlich ist es auch möglich, dass das Stabilisierungselement auf der Innenseite des Schlauches festgelegt wird, beispielsweise indem zunächst das Stabilisierungselement auf die Gliedmaße aufgelegt und anschließend der Schlauch darübergezogen wird. Muss das Körpergelenk nicht mehr über das Stabilisierungselement gestützt bzw, in einem geringeren Maß immobilisiert werden, wird das Stabilisierungselement nicht mehr angelegt, so dass die Stützwirkung nur noch von dem Schlauch ausgeübt wird. Die Bandage kann somit je nach Bedarf abgerüstet oder aufgerüstet werden.

Statt einer Anbringung des Stabilisierungselementes an der Oberfläche des Schlauches kann auch vorgesehen sein, dass in oder an dem Schlauch eine Tasche zur Aufnahme des Stabilisierungselementes angeordnet ist, in die das Stabilisierungselement eingesteckt wird, wenn das Gelenk in zumindest einer Bewegungsrichtung in seiner Beweglichkeit eingeschränkt werden soll. Die Tasche kann auf der Innen- oder Außenseite des Schlauches angeordnet oder ausgebildet sein, so dass das Stabilisierungselement einfach eingeschoben und herausgezogen werden kann.

Bei herkömmlichen Bandagen dient die Polsterung gleichzeitig als Biegesteifigkeitserhöhungselement, das die Bewegung des Handgelenks einschränkt. In der erfindungsgemäßen Bandage ist das Stabilisierungselement, das auch als Biegefestigkeitserhöhungselement bezeichnet werden könnte, zum Fixieren, z.B. von außen oder durch Einschieben in eine Tasche, an der Knochenvorsprungstelle ausgebildet. Das heißt in anderen Worten, dass es zum reversiblen Fixieren an der angelegten Bandage ausgebildet ist. Es ist damit möglich, zunächst die Bandage überzustreifen und erst danach das Stabilisierungselement zu positionieren und zu fixieren. So wird beispielsweise das Handgelenk sicher in einer Schonhaltung gehalten. Gleichzeitig ermöglicht das extern angeordnete oder einschiebbare Stabilisierungselement ein einfaches An- und Ausziehen der Bandage.

Bevorzugt weist die Bandage kompressionsreduzierte Ränder bzw. Bündchen auf, um das Anziehen der Bandage zu erleichtern.

Unter dem Merkmal, dass der Schlauch an der Knochenvorsprungstelle eine geringere Kompressionswirkung aufweist als außerhalb bzw. jenseits der Knochenvorsprungstelle, ist insbesondere zu verstehen, dass der Schlauch so ausgebildet ist, dass er an der Knochenvorsprungstelle einen kleineren Druck auf das Körperglied ausübt.

Gemäß einer bevorzugten Ausführungsform ist das Stabilisierungselement über eine lösbare, einstellbare Schnellverschlussvorrichtung mit dem Schlauch verbunden. Eine derartige Schnellverschlussvorrichtung kann beispielsweise ein Streifen aus Stoff sein, der über zumindest eine, bevorzugt aber zwei Klettverbindungen mit dem Schlauch verbindbar ist. Beispielsweise ist es möglich, dass die Schnellverschlussvorrichtung mit einem ersten Klettelement am Schlauch befestigbar ist. Über das erste Klettelement ist so die Position des Stabilisierungselements relativ zum Schlauch festlegbar. Die Schnellverschlussvorrichtung kann zudem ein zweites Klettelement besitzen, das ein Befestigen am Schlauch oder ein Befestigen an der Schnellverschlussvorrichtung selbst erlaubt. Durch Lösen des zweiten Klettelements kann das Stabilisierungselement von einer ersten Stellung, in der das Stabilisierungselement fest am Schlauch befestigt ist, in eine zweite Stellung gebracht werden, in der das Stabilisierungselement nur über das erste Klettelement am Schlauch befestigt ist. Beim Anziehen der Bandage ist das Stabilisierungselement lose am Schlauch befestigt oder vom Schlauch getrennt und nach dem Überstreifen des Schlauchs wird das Stabilisierungselement durch das erste und das zweite Klettelement fest am Schlauch befestigt, so dass Körpergelenk geschützt wird.

Bevorzugt besitzt der Schlauch an der Knochenvorsprungstelle eine erhöhte Flächenelastizität. Durch die erhöhte Flächenelastizität übt der Schlauch an der Knochenvorsprungstelle einen verringerten Druck aus. Das heißt, der Schlauch gibt an der Knochenvorsprungstelle leichter nach. Dadurch kann die Bandage ausnehmungsfrei ausgebildet werden, was den Vorteil hat, dass sie besonders einfach anzuziehen ist. Die Bandage ist dadurch auch besonders einfach zu fertigen und besitzt keine auftragenden Nähte.

Alternativ oder additiv kann die Bandage eine Ausnehmung besitzen, was jedoch gegenüber einer erhöhten Flächenelastizität weniger vorteilhaft ist.

In einer bevorzugten Ausführungsform ist das Stabilisierungselement zum reversiblen Fixieren, von außen an der Knochenvorsprungstelle, insbesondere zum reversiblen Vorspannen von außen gegen die Knochenvorsprungstelle mittels der Schnellverschlussvorrichtung, ausgebildet. Auf diese Weise kann das Stabilisierungselement beim An- und Ausziehen der Bandage besonders leicht von seiner ersten Stellung, in der es fest am Schlauch anliegt, in seine zweite Stellung, in der es lose am Schlauch befestigt ist oder vom Schlauch getrennt ist, gebracht werden.

Eine besonders angenehm zu tragende Bandage wird erhalten, wenn das Stabilisierungselement eine Pelotte ist.

Eine besonders einfach an- und auszuziehende Bandage wird erhalten, wenn der Schlauch zumindest in einer Umgebung der Knochenvorsprungstelle ungepolstert ist. Polster weisen in der Regel eine höhere Biegesteifigkeit als ein Gestrick auf und sind zudem meist weniger elastisch. Beides erschwert ein Anund Ausziehen. Bei Bandagen nach dem Stand der Technik werden Polsterungen verwendet, um die Knochenvorsprungstelle von der Kompression des Schlauchs zu entlasten. Bei der erfindungsgemäßen Bandage kann hierauf jedoch verzichtet werden, da das Stabilisierungselement kein integraler Bestandteil des Schlauchs ist, sondern von diesem lösbar ausgebildet ist.

In einer bevorzugten Ausführungsform besitzt das Stabilisierungselement ein Trageelement und eine dem Schlauch zugewandte Polsterung des Trageelements, wobei das Trageelement in einer Wirkkraftrichtung der Bandage eine höhere Biegesteifigkeit aufweist als in einer senkrecht zur Längsrichtung verlaufenden Querrichtung. Dadurch wird das Stabilisierungselement, beispielsweise die Pelotte, autoadaptiv und hat zudem eine verbesserte Brückenwirkung, die das Körpergelenk stabilisiert. Die Wirkkraftrichtung ist diejenige Richtung, in die sich das zu stützende Körperglied nicht bewegen soll. Das heißt, die Bandage ist ausgebildet, um ein Teil des Körpergelenks in einer Bewegung in Wirkkraftrichtung zu hemmen. Ist die Bandage eine Handgelenkbandage, so ist die Wirkkraftrichtung entweder radial-ulnar und/oder volar-dorsal, wobei die Einschränkung oder Behinderung der Bewegung beispielsweise volar-dorsal und ulnar durch eine rinnenartige Ausgestaltung des Stabilisierungselementes, das sich über die Elle, das Handgelenk und die Handkante erstreckt, erreicht werden kann.

Das Trageelement kann, um eine in Längsrichtung höhere Biegesteifigkeit aufzuweisen, beispielsweise in Längsrichtung verlaufende Schlitze, in Längsrichtung angeordnete Löcher oder in Längsrichtung verlaufende Materialverjüngungen aufweisen. Diese haben die Wirkung einer in Längsrichtung verlaufenden Rippung.

Besonders bevorzugt ist die Bandage als Handgelenkbandage ausgebildet und besitzt eine Daumenöffnung, wobei die Knochenvorsprungstelle bei Benutzung am Griffelfortsatz anliegt. In anderen Worten handelt es sich dann um eine Handgelenkbandage mit einem elastischen Schlauch aus Gestrick, der ausgebildet ist, um um ein Handgelenk herum eng anzuliegen und der mindestens zwei Öffnungen zum Durchstecken einer Hand aufweist, wobei der Schlauch an einer Griffelfortsatzstelle, die bei Benutzung der Handgelenkbandage an einem Griffelfortsatz anliegt, eine geringere Kompressionswirkung aufweist als jenseits der Griffelfortsatzstelle, wobei die Handgelenkbandage ein Stabilisierungselement, das zum Fixieren einer vorgebbaren Position am Schlauch ausgebildet ist, umfasst. Das vorzugsweise gepolsterte Stabilisierungselement kann eine Ausnehmung zum teilweisen Aufnehmen des Griffelfortsatzes aufweisen und ist auf den Griffelfortsatz vorspannbar am Schlauch befestigbar. Das Stabilisierungselement ist beispielsweise ausgebildet, um lateral bezüglich des Handgelenks im Bereich des Griffelfortsatzes angeordnet zu werden und um das Handgelenk zu überbrücken, so dass es einerseits an der Elle und andererseits an der lateralen Handoberseite anliegt.

Alternativ ist die Bandage als Kniebandage ausgebildet, wobei die Knochenvorsprungstelle bei Benutzung an der Kniescheibe oder an den Kondylen anliegt.

Um eine besonders einfach zu fertigende Bandage zu erhalten, kann das Gestrick ein einlagiges Gestrick sein. An der Knochenvorsprungstelle kann das Gestrick dann schussfadenfrei oder schussfadenreduziert sein, so dass das Gestrick eine höhere Flächenelastizität aufweist. Jenseits der Knochenvorsprungstelle besitzt das Gestrick vorzugsweise einen Schussfaden bzw. eine höhere Schussfadendichte, was zu einer geringeren Flächenelastizität und damit zu einer erhöhten Kompression führt.

Auftragende Nähte werden vermieden, wenn die Bandage taschenfrei ist und/oder rundgestrickt ist.

Im Folgenden werden exemplarische Ausführungsbeispiele der Erfindung anhand der beigefügten Zeichnungen näher erläutert. Dabei zeigen
- Figur 1: eine erfindungsgemäße Bandage, die an einem Handgelenk anliegt, ohne Stabilisierungselement;
- Figur 2: die Bandage gemäß Figur 1 mit angelegtem Stabilisierungselement;
- Figur 3: die angelegte Bandage in einer weiteren perspektivischen Ansicht;
- Figuren 4a-4c: Stabilisierungselemente der erfindungsgemäßen Bandage;
- Figur 5: eine alternative Ausgestaltung der Bandage;
- Figur 6: eine dorsale Draufsicht auf eine Bandage gemäß Fig. 5; sowie
- Figur 7: eine Variante der Bandage gemäß Fig. 5.

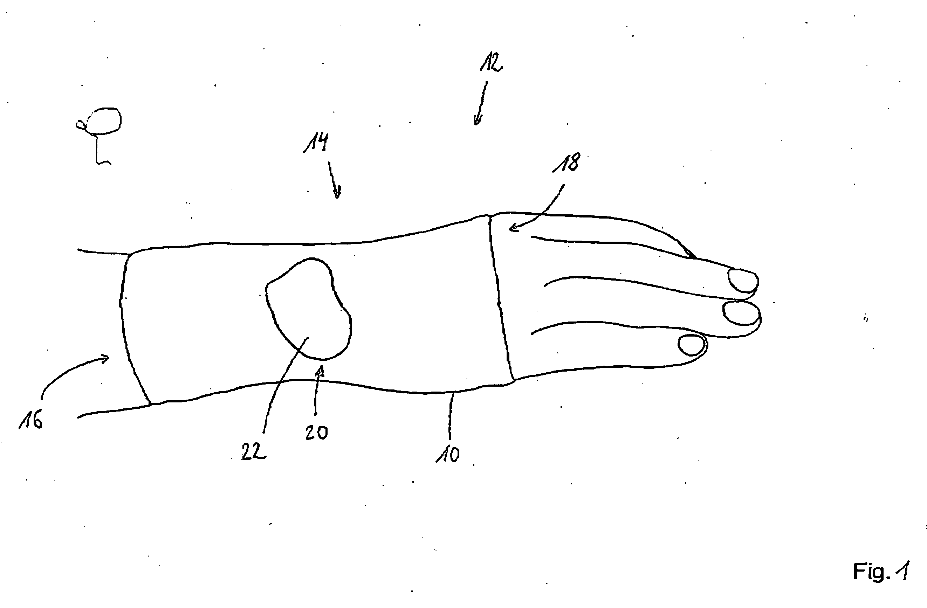

Figur 1 zeigt einen elastischen Schlauch 10 einer Handgelenkbandage 12, der über ein Körpergelenk in Form eines Handgelenks 14 eines Patienten gezogen ist. Der elastische Schlauch 10 weist eine proximale Öffnung 16 und eine distale Öffnung 18 auf, durch die das Körpergelenk hindurchgesteckt wird.

Am Handgelenk 14 befindet sich ein Griffelfortsatz 20, der in Figur 1 von einer Knochenvorsprungstelle 22 des elastischen Schlauchs 10 abgedeckt ist. Die Knochenvorsprungstelle 22 kann, da es sich um eine Handgelenkbandage handelt, auch als Griffelfortsatzstelle des elastischen Schlauchs 10 bezeichnet werden und bildet einen Teil eines Gestricks, aus dem der Schlauch 10 aufgebaut ist.

Jenseits der Knochenvorsprungstelle 22 erstreckt sich ein Grundkörper 24 des Schlauchs 10. Die Knochenvorsprungstelle 22 und der Grundkörper 24 sind einstückig durch Flach- oder Rundstricken hergestellt und unterscheiden sich dadurch, dass das Gestrick im Grundkörper 24 eine höhere Schussfadendichte und/oder hinterlegte kompressionserhöhende Fäden besitzt, das Gestrick an der Knochenvorsprungstelle hingegen eine kleinere Schussfadendichte. Dadurch ist die Flächenelastizität des Grundkörpers 24 geringer als die der Knochenvorsprungstelle. In anderen Worten weist die Knochenvorsprungstelle 22 eine geringe Kompressionswirkung auf als der Grundkörper 24. Das bedeutet, dass die Knochenvorsprungstelle 22 einem Eindringen beispielsweise einer Stahlkugel mit 1 mm Durchmesser um einen Millimeter Tiefe einen beispielsweise um Faktor zwei geringeren Widerstand entgegensetzt als das Gestrick des Grundkörpers 24. Aus diesem Grund übt die Handgelenkbandage 12 am Griffelfortsatz 20 eine kleinere Kraft auf die Hand aus als jenseits der Knochenvorsprungstelle im Grundkörper 24.

Die Knochenvorsplungstelle hat einen Querschnitt von beispielsweise weniger als 6 cm Durchmesser, so dass trotz der Knochenvorsprungstelle 22 der elastische Schlauch 10 eine hinreichend große Gesamt-Kompression auf das Handgelenk 14 aufbringen kann.

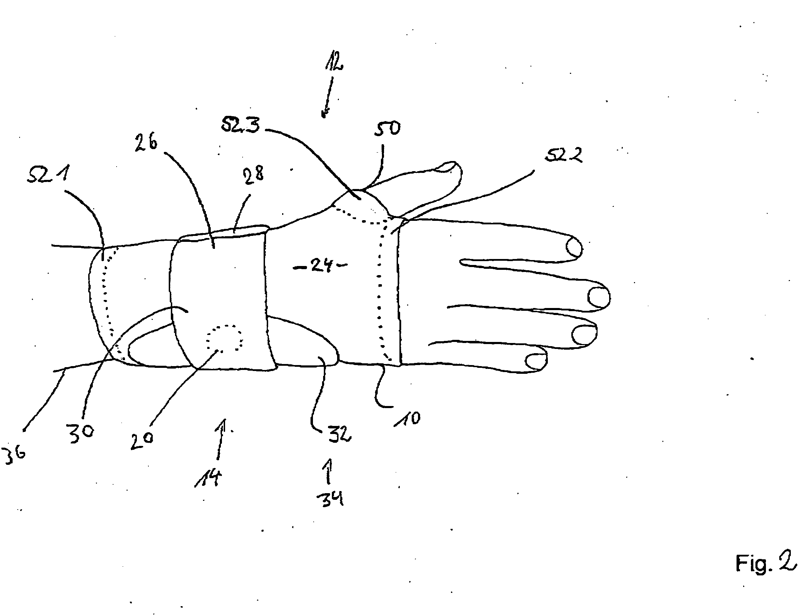

Figur 2 zeigt die Handgelenkbandage 12, die eine Schnellverschlussvorrichtung 26 umfasst, die im vorliegenden Fall durch ein elastisches Band gebildet ist, das an einem seiner Enden ein schematisch eingezeichnetes erstes Klettelement 28 besitzt, mit dem die Schnellschlussvorrichtung 26 am Schlauch 10 in Kletteingriff bringbar und so befestigbar ist. An ihrem gegenüberliegenden Ende besitzt die Schnellschlussvorrichtung 26 ein nicht eingezeichnetes zweites Klettelement. Zwischen beiden Klettelementen ist ein Streifen 30 aus Flauschband angeordnet.

Die Schnellverschlussvorrichtung 26 ist im Bereich des Handgelenks 14 angeordnet und fixiert ein Stabilisierungselement 32 relativ zum Schlauch 10. Das Stabilisierungselement 32 ist an dem Streifen 30 angeklettet. Alternativ oder additiv ist das Stabilisierungselement 32 am elastischen Schlauch 10 lösbar befestigt, beispielsweise angeklettet. Damit kann das Stabilisierungselement 32 zum leichten An- und Ausziehen der Handgelenkbandage 12 vom Schlauch 10 abgetrennt werden.

Das Stabilisierungselement 32 hat eine Länge L, die so gewählt ist, dass es sich von einer Handmitte 34 über den Griffelfortsatz 20, der gestrichelt eingezeichnet ist, bis zu einer Elle 36 erstreckt. Dadurch überbrückt das Stabilisierungselement 32 das Handgelenk 14 und warnt einen Benutzer der Bandage 12, wenn er das Handgelenk 14 ulnar zu weit schwenken will.

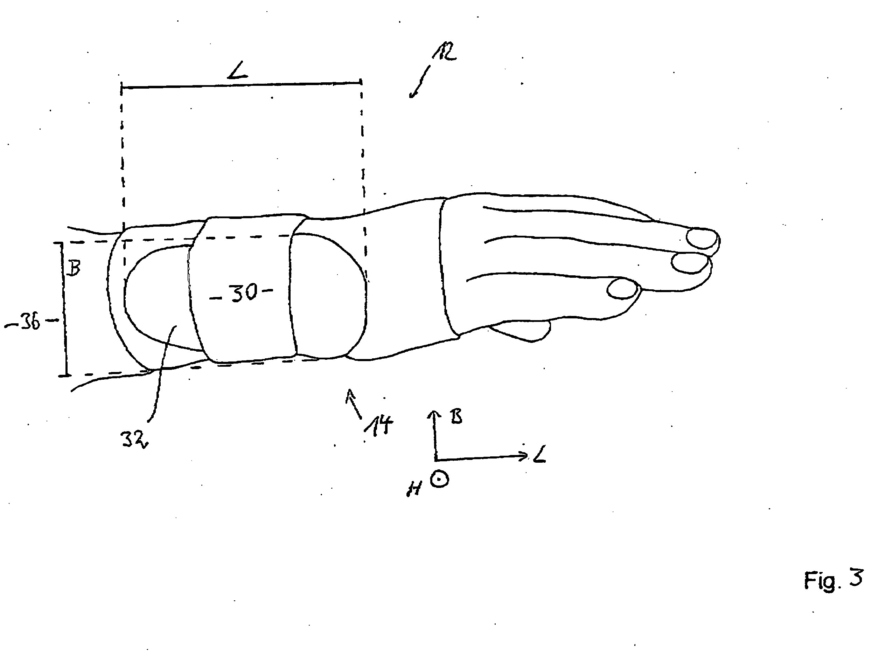

Figur 3 zeigt eine zweite perspektivische Ansicht der Handgelenkbandage 12 gemäß Figur 2. Es ist zu erkennen, dass sich das Stabilisierungselement 32 im Wesentlichen über eine Höhe H, die einer Höhe des Handgelenks 14 in seiner seitlichen Projektion entspricht, erstreckt. Dabei hat das Stabilisierungselement 32 eine Länge L, die größer ist als eine Breite B. Beispielsweise ist die Länge L das 1,5 bis 2-fache der Breite B.

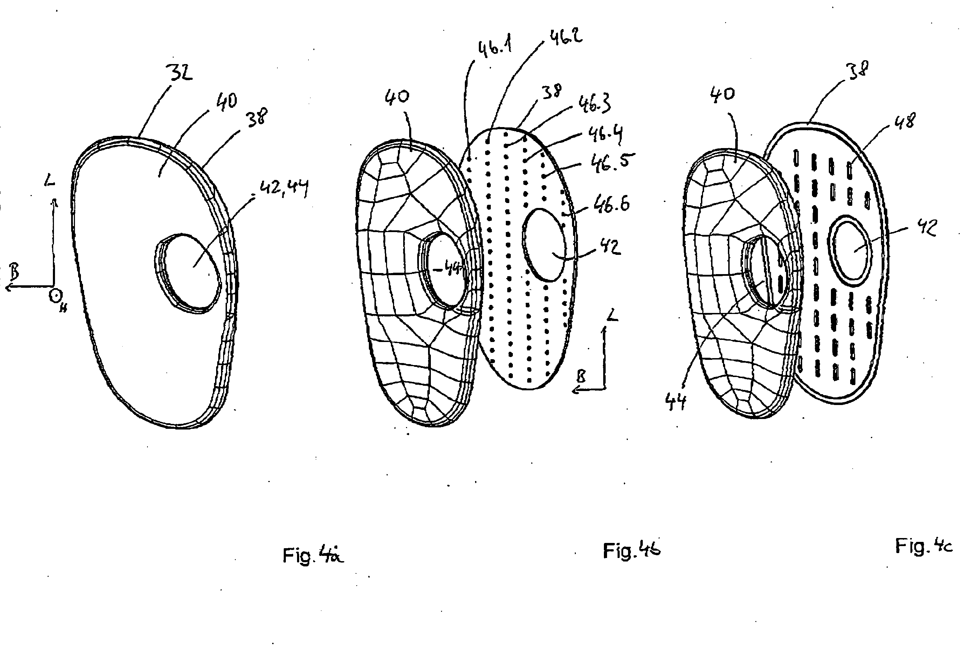

Figur 4a zeigt das Stabilisierungselement 32, das ein Trageelement 38 und eine Polsterung 40 umfasst. Das Trageelement 38 weist eine Ausnehmung 42 auf, die mit einer Ausnehmung 44 der Polsterung überein liegt. Die Ausnehmungen 42, 44 sind so angeordnet, dass sie bei Einsatz der Handgelenksbandage über dem Griffelfortsatz liegen. Das Stabilisierungselement 32 ist zudem so geformt, dass es dann, wenn die Ausnehmung 42, 44 über dem Griffelfortsatz liegen, sich dem Handgelenk anpasst.

Figur 4b zeigt eine Ausführungsform des Trageelements 38, die in Reihen 46.1, 46.2), ... angeordnete Löcher aufweist. Dadurch besitzt das Trageelement 38 in der Längsrichtung L eine höhere Biegesteifigkeit als senkrecht zur Längsrichtung, nämlich in einer Querrichtung B.

Figur 4 c zeigt eine alternative Ausführungsform des Trageelements 38, das in Reihen angeordnete Schlitze 48 zum gleichen Zweck aufweist. Die Stabilisierungselemente 32 sind mit Flauschstoff umgeben, so dass sie über einen Klettverbinder am Schlauch 10 befestigbar sind.

Figur 2 zeigt, dass der Schlauch 10 eine Daumenöffnung aufweist. Der Schlauch 10 besitzt zudem um die proximale Öffnung 16, die distale Öffnung 18 und das Daumenöffnung 50 durch eine Strichelung gekennzeichnete Bündchen 52.1., 52.2 und 52.3. In diesem Bündchen 52 übt der Schlauch 10 eine geringere Kompressionswirkung auf die Hand aus als im Rest des Grundkörpers 24.

Die Handgelenkbandage 12 wird beispielsweise durch Flach- oder Rundstricken hergestellt. Zum Erhöhen der Kompressionswirkung kann in das Gestrick, das auch ein Gewirk sein kann, ein Schussfaden oder zumindest ein kompressionserhöhender Faden eingearbeitet sein. Da die Handgelenksbandage 12 taschenfrei und ausnehmungsfrei ist, sind auftragende Nähte entbehrlich.

Zum Bedienen der Handgelenksbandage 12 führt der Benutzer zunächst wie in Figur 1 gezeigt, seine Hand durch den elastischen Schlauch 10 ein. Anschließend legt er, wie in Figur 2 gezeigt, das Stabilisierungselement 32 an und fixiert dieses mit der Schnellverschlussvorrichtung 26. Die Schnellverschlussvorrichtung 26 ist dann in ihrer ersten Stellung.

Beim Ausziehen hat der Benutzer die Wahl, ob er das Stabilisierungselement 32 am Schlauch 10 fixiert lässt oder ob er es zum Ausziehen abnimmt. Alternativ kann er die Schnellschlussvorrichtung nur in ihrem zweiten Klettelement lösen, so dass sie nur noch mit ihrem ersten Klettelement 28 (vgl. Figur 2) am Schlauch befestigt ist. Die Handgelenkbandage 10 ist dann einfach abstreifbar.

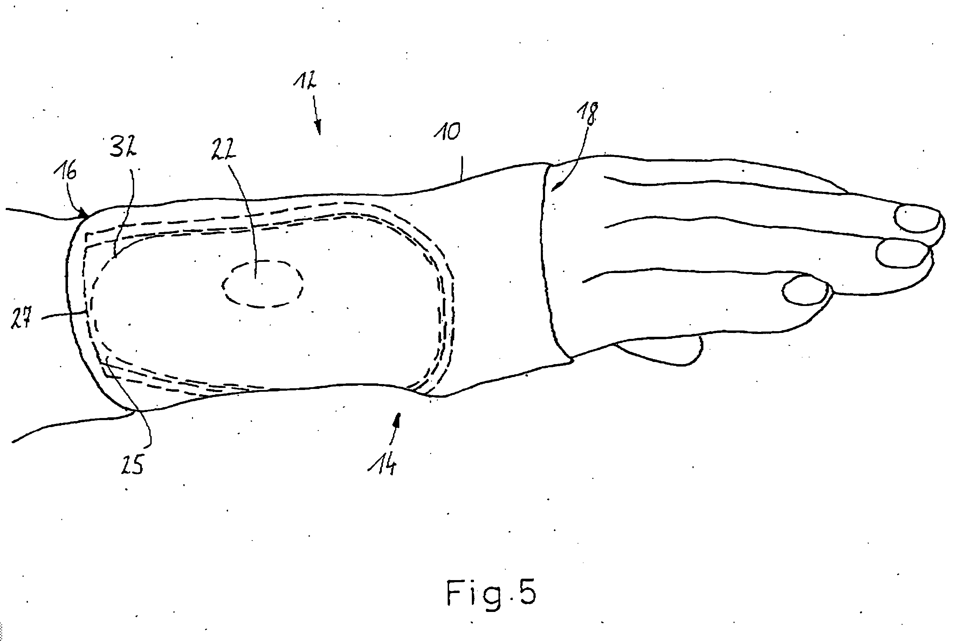

Figur 5 zeigt eine erfindungsgemäße Kniebandage 54 mit dem elastischen Schlauch 10 aus Gestrick, der ausgebildet ist, um an einem Knie 56 eng anzuliegen. Bei der Kniebandage 54 weist der Schlauch 10 an einer Knochenvorsprungstellen 22.1, die an einer Patella 58 anliegt, eine geringere Kompressionswirkung auf als außerhalb der Knochenvorsprungstelle 22.1. Dazu hat die Kniebandage 54, wie oben für die Handgelenkbandage beschrieben, an der Knochenvorsprungstelle 22.1 eine erhöhte Flächenelastizität.

Die Kniebandage 54 besitzt zudem eine weitere Knochenvorsprungstelle 22.2, die an einem Fibularköpfchen 60 anliegt, und weist dort ebenfalls eine geringere Kompressionswirkung auf als außerhalb der Knochenvorsprungstelle 22.2. Beispielsweise entspricht die Kompressionswirkung in der Knochenvorsprungstelle 22.2 der Kompressionswirkung in der Knochenvorsprungstelle 22.1.

Zusätzlich umfasst die Kniebandage 54 ein nicht eingezeichnetes Stabilisierungselement, das zum Fixieren von außen an der Knochenvorsprungstelle 22.1 und/oder 22.2 ausgebildet ist. Das Stabilisierungselement kann beispielsweise lateral, medial, proximal oder distal an der Patella 58 anliegen und auf diese eine Kraft ausüben. Alternativ ist das Stabilisierungselement ausgebildet, um die Patella 58 ringförmig oder C-förmig zu umgeben.

In der Figur 5 ist eine Variante einer Handgelenkbandage 12 mit einem elastischen Schlauch 10 aus einem Gestrick mit einer proximalen Öffnung 16 und einer distalen Öffnung 18 gezeigt. Der grundsätzliche Aufbau entspricht dem Aufbau der Bandage 12 gemäß der Figuren 1 bis 3, jedoch mit dem Unterschied, dass statt einer außenseitigen Festlegung des Stabilisierungselementes 32 in dem Schlauch 10 eine Tasche 25 ausgebildet ist, in die das Stabilisierungselement 32 hineingeschoben werden kann. Dazu ist bevorzugt im Bereich der proximalen Öffnung 16 eine Einschuböffnung 27 vorgesehen, durch die das Stabilisierungselement 32, das beispielsweise als eine rinnenartige Kunststoffschiene ausgebildet sein kann, eingeschoben werden kann. Im Bereich der Auswölbung des distalen Endes der Elle ist der Bereich mit einer geringeren Kompressionswirkung ausgebildet, so dass im angelegten Zustand der Bandage 12 dieser Bereich 22 als Knochenvorsprungsstelle 22 ausgebildet ist. Das Stabilisierungselement 32 weist an der korrespondierenden Stelle eine nicht dargestellte Ausnehmung auf, um zu vermeiden, dass durch das Stabilisierungselement 32 im angelegten Zustand ein übermäßiger Druck auf die vorspringende Knochenstruktur, insbesondere den Griffelfortsatz 20, ausgeübt wird. Die Tasche 25 kann von außen auf den Schlauch 10 aufgebracht und daran festgelegt sein. Die Festlegung erfolgt beispielsweise über ein Vernähen, Verkleben oder Verschweißen eines entsprechend elastisch ausgebildeten Stoffelementes auf dem Schlauch 10. Alternativ kann die Tasche 25 innenseitig an dem Schlauch 10 angebracht sein, so dass die Bandage 12 eine glattflächige äußere Oberfläche aufweist, während auf der Innenseite im Einschubbereich eine weitere Gewebelage angeordnet ist. Das Gewebe der Tasche 25 kann von dem Schlauchgewebe verschieden sein. Die Zugangsöffnung 27 der Tasche kann verschließbar ausgebildet sein, um das Stabilisierungselement 32 innerhalb der Tasche 25 fixiert zu halten. Die Verschlusseinrichtung ist bevorzugt als ein Klettverschluss ausgebildet.

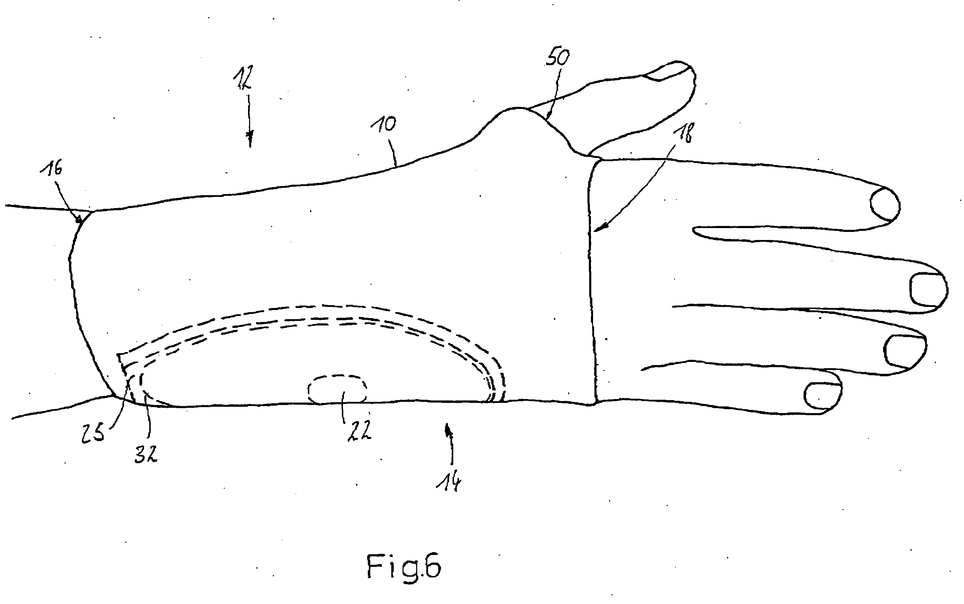

Grundsätzlich ist es auch möglich, dass die Tasche 25 einstückig in dem Schlauch 12 eingearbeitet oder bei einer doppelwandigen Ausgestaltung des Schlauches 10 durch Trennnähte ausgebildet wird. In der Figur 6 ist eine Dorsaldraufsicht der Ausgestaltung gemäß der Figur 5 gezeigt. Auch bei einer Ausgestaltung der Bandage 12 mit dem Schlauch 10 und einer innenseitig angeordneten Tasche 25 besteht der Vorteil, dass das Stabilisierungselement 32 nach dem Anlegen des Schlauches 10 um das Handgelenk 14 eingeführt werden kann, wodurch das Anlegen der Bandage 12 wesentlich erleichtert wird. Darüber hinaus besteht die Möglichkeit, dass die Bandage 12 an den Gesundungsfortschritt oder an die medizinische Indikation angepasst werden kann, indem das Stabilisierungselement 32 von der Bandage 12 entfernt wird, wenn es nicht mehr benötigt wird.

In dem Ausführungsbeispiel gemäß der Figuren 5 und 6 ist das Stabilisierungselement 32 an der äußeren Seite des Handgelenkes 14 angeordnet, also ulnar. Das Stabilisierungselement 32 stützt sich dabei an den Unterarm im Bereich der Elle ab, sowie ulnar im Bereich des Handgelenks sowie teilweise an dem Mittelhandknochen bzw. entlang der Handkante. Das Stabilisierungselement 22 kann dabei rinnenartig ausgebildet sein, so dass eine Bewegung der Hand ulnar sowie eine Dorsal- und Volarbewegung eingeschränkt wird. Je weiter die Überdeckung des Stabilisierungselementes 32 über das Handgelenk ausgebildet ist, desto größer ist der Widerstand insbesondere gegen eine Volar-Dorsal-Bewegung und eine Ulnarbewegung der Hand. Je rigider das Stabilisieningselement 32 ausgebildet ist, desto höher wird der Widerstand in die Wirkrichtung.

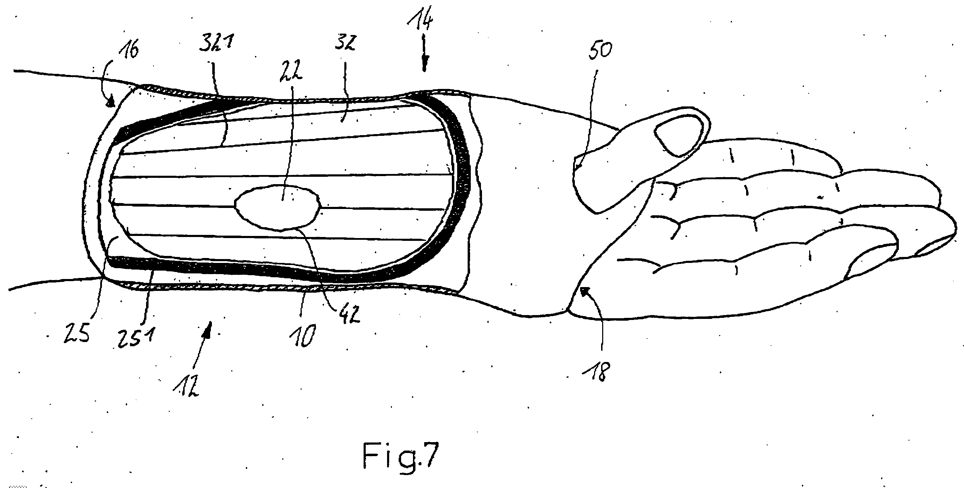

In der Figur 7 ist eine Variante der Erfindung gezeigt, bei der die Tasche 25 radial angeordnet ist, so dass die Knochenvorsprungstelle 22 des Schlauches 10 im Bereich des distalen Endes der Speiche liegt. Dementsprechend ist die Tasche 25 so ausgebildet, dass das Stabilisierungselement 32 das Handgelenk 14 innen, also radial abstützt, so dass eine Radialverschwenkung der Hand behindert oder verhindert wird. Stützt sich das Stabilisierungselement 32 an der Handinnenfläche ab, wird nur eine Volarflexion verhindert, eine Dorsalflexion der Hand bleibt im wesentlichen uneingeschränkt, beeinträchtigt nur durch die Elastizität des Schlauches.

Die Tasche 25 kann eingearbeitet, angestrickt oder durch Abnähen ausgebildet sein. In der teilgeschnittenen Figur 7 ist zu erkennen, dass das Gestrick 10, das der Haut zugewandt ist, die Knochenvorsprungstelle 22 mit dem verringerten Kompressionsvermögen aufweist, so dass an dieser Stelle nur ein verringerter Druck auf das Gewebe und den Knochen ausgeübt wird. Ebenfalls ist eine Schweiß- oder Klebefläche 251 zu erkennen, entlang der das Taschengewebe angeklebt oder angeschweißt werden kann. Das Stabilisierungselement 32 ist plattenartig ausgebildet und weist Materialschwächungen, Eindrückungen oder dergleichen entlang der gewünschten Biegelinien 321 auf, so dass sich das Stabilisierungselement 32 um seine Längsachse biegen und um das Handgelenk herum legen lässt, Eine Bewegung um eine Achse senkrecht zu der Längserstreckung des Stabilisierungselementes 32 wird verhindert oder zumindest behindert, da quer zur Längserstreckung keine Sollbieglinien oder Materialschwächungen ausgebildet sind und durch die Biegung um die Längsachse eine erhöhte Biegesteifigkeit erzielt wird. In der Figur 7 ist ebenfalls die Ausnehmung 42 in dem Stabilisierungselement 32 gezeigt , die oberhalb der Knochenvorsprungstelle 22 angeordnet ist.

### Bezugszeichenliste

- 10: elastischer Schlauch
- 12: Handgelenkbandage
- 14: Handgelenk
- 16: proximal Öffnung
- 18: distale Öffnung
- 20: Griffelfortsatz
- 22: Knochenvorsprungstelle
- 24: Grundkörper
- 26: Schnellverschlussvorrichtung
- 28: erstes Klettelement
- 30: Streifen
- 32: Stabilisierungselement
- 34: Handmitte
- 36: Elle
- 38: Trageelement
- 40: Polsterung
- 42: Ausnehmung
- 44: Ausnehmung
- 46: Reihe
- 48: Schlitz
- 50: Daumenöffnung
- 52: Bündchen
- 54: Kniebandage
- 56: Knie
- 58: Patella
- 60: Fibularköpfchen

- L: Länge
- B: Breite

## Patentansprüche

1. Bandage mit einem elastischen Schlauch (10) aus Gestrick, der ausgebildet ist, um an einem Körpergelenk (14) eng anzuliegen, wobei der Schlauch (10) an einer Knochenvorsprungstelle (22), die bei Benutzung der Bandage an einem Knochenvorsprung (20) anliegt, eine geringere Kompressionswirkung aufweist als außerhalb der Knochenvorsprungstelle (22), **dadurch gekennzeichnet, dass** die Bandage (12) ein Stabilisierungselement (32), das zum Fixieren an der Knochenvorsprungstelle (22) ausgebildet ist, umfasst, das das Körpergelenk (14) zumindest teilweise überbrückt, um die Bewegung des Körpergelenks (14) in zumindest einer Richtung zu hemmen, wobei das Stabilisierungselement (32) eine Ausnehmung aufweist, die in ihren Abmessungen im Wesentlichen dem Knochenvorsprung (20) entspricht.

2. Bandage nach Anspruch 1, **dadurch gekennzeichnet, dass** das Stabilisierungselement (32) über eine lösbare, einstellbare Schnellverschlussvorrichtung (26) mit dem Schlauch (10) verbunden ist.

3. Bandage nach Anspruch 1 oder 2, **dadurch gekennzeichnet, dass** das Stabilisierungselement (32) an der Oberfläche des Schlauches (10), insbesondere von außen an dem Schlauch (10) befestigt ist.

4. Bandage nach Anspruch 1 oder 2, **dadurch gekennzeichnet, dass** in oder an dem Schlauch (10) eine Tasche (25) zur Aufnahme des Stabilisierungselementes (32) angeordnet ist.

5. Bandage nach einem der vorstehenden Ansprüche, **dadurch gekennzeichnet, dass** der Schlauch (10) an der Knochenvorsprungstelle (22) eine erhöhte Flächenelastizität besitzt.

6. Bandage nach einem der vorstehenden Ansprüche, **dadurch gekennzeichnet, dass** das Stabilisierungselement (32) zum reversiblen Fixieren von außen um die Knochenvorsprungstelle (22), insbesondere zum reversiblen Vorspannen von außen gegen die Knochenvorsprungstelle (22), ausgebildet ist.

7. Bandage nach einem der vorstehenden Ansprüche, **dadurch gekennzeichnet, dass** das Stabilisierungselement (32) eine Pelotte ist.

8. Bandage nach einem der vorstehenden Ansprüche, **dadurch gekennzeichnet, dass** der Schlauch (10) zumindest in einer Umgebung der Knochenvorsprungstelle (22) ungepolstert ist.

9. Bandage nach einem der vorstehenden Ansprüche, **dadurch gekennzeichnet, dass** das Stabilisierungselement (32)
- ein Trageelement (38) und
- eine dem Schlauch (10) zugewandte Polsterung (40) des Trageelements (38) aufweist,
- wobei das Tragelement (38) in einer Wirkkraftrichtung, insbesondere in einer Längsrichtung (L), eine höhere Biegesteifigkeit aufweist als in einer senkrecht zur Wirkkraftrichtung verlaufenden Querrichtung (B).

10. Bandage nach einem der vorstehenden Ansprüche, **dadurch gekennzeichnet, dass** sie als Handgelenkbandage (12) ausgebildet ist und eine Daumenöffnung (50) besitzt, wobei die Knochenvorsprungstelle (22) bei Benutzung am Griffelfortsatz (20) anliegt.

11. Bandage nach einem der Ansprüche 1 bis 9, **dadurch gekennzeichnet, dass** sie als Kniebandage ausgebildet ist, wobei die Knochenvorsprungstelle an der Patella und/oder an den Kondylen anliegt.

## Claims

1. Bandage having an elastic hose (10) made of a knit, which is designed to rest closely against a joint (14) of the body, wherein the hose (10) has a lesser compression effect at a bony-projection area (22), which rests against a bony projection (20) when the bandage is used, than away from the bony projection area (22), **characterized in that** the bandage (12) comprises a stabilizing element (32) which is designed to be fixed at the bony-protection area (22) and which at least partially bridges the joint (14) of the body in order to restrict the movement of the joint (14) of the body in at least one direction, wherein the stabilizing element (32) has a cutout, the dimensions of which correspond substantially to the bony projection (20).

2. Bandage according to Claim 1, **characterized in that** the stabilizing element (32) is connected to the hose (10) via a releasable, settable quickacting closure device (26).

3. Bandage according to Claim 1 or 2, **characterized in that** the stabilizing element (32) is fastened to the surface of the hose (10), and in particular is fastened to the hose (10) from the outside.

4. Bandage according to Claim 1 or 2, **characterized in that** a pocket (25) for receiving the stabilizing element (32) is arranged in or on the hose (10).

5. Bandage according to one of the preceding claims, **characterized in that** the hose (10) has increased surface elasticity in the bony-projection area (22).

6. Bandage according to one of the preceding claims, **characterized in that** the stabilizing element (32) is designed to be reversibly fixed from the outside about the bony-projection area (22), in particular to be reversibly pretensioned from the outside against the bony-projection area (22).

7. Bandage according to one of the preceding claims, **characterized in that** the stabilizing element (32) is a pad.

8. Bandage according to one of the preceding claims, **characterized in that** the hose (10) is not padded at least in the vicinity of the bony-projection area (22).

9. Bandage according to one of the preceding claims, **characterized in that** the stabilizing element (32)
- has a support element (38) and
- padding (40), facing the hose (10), of the support element (38),
- wherein the support element (38) has greater flexural rigidity in one active-force direction, in particular in a longitudinal direction (L), than in a transverse direction (B) extending perpendicularly to the active-force direction.

10. Bandage according to one of the preceding claims, **characterized in that** it is designed as a wrist bandage (12) and has a thumb opening (50), wherein the bony-projection area (22) rests against the styloid process (20) in use.

11. Bandage according to one of Claims 1 to 9, **characterized in that** it is designed as a knee bandage, wherein the bony-projection area rests against the patella and or against the condyles.

## Revendications

1. Bandage avec un tuyau élastique (10) en tricot, qui est réalisé pour être appliqué étroitement sur une articulation corporelle (14), dans lequel le tuyau (10) présente au niveau d'un emplacement pour saillie osseuse (22), qui s'applique lors de l'utilisation du bandage contre une saillie osseuse (20), un effet de compression plus faible qu'à l'extérieur de l'emplacement pour saillie osseuse (22), **caractérisé en ce que** le bandage (12) inclut un élément de stabilisation (32) qui est réalisé pour la fixation au niveau de l'emplacement pour saillie osseuse (22), élément de stabilisation qui fait au moins partiellement un pontage de l'articulation corporelle (14) afin d'entraver le mouvement de l'articulation corporelle (14) dans au moins une direction, et l'élément de stabilisation (32) comporte un évidement qui correspond quant à ses dimensions sensiblement à la saillie osseuse (20).

2. Bandage selon la revendication 1, **caractérisé en ce que** l'élément de stabilisation (32) est relié avec le tuyau (10) via un dispositif de fermeture rapide (26) détachable et réglable.

3. Bandage selon la revendication 1 ou 2, **caractérisé en ce que** l'élément de stabilisation (32) est fixé à la surface du tuyau (10) et en particulier sur le tuyau (10) depuis l'extérieur.

4. Bandage selon la revendication 1 ou 2, **caractérisé en ce qu'**une poche (25) destinée à recevoir l'élément de stabilisation (32) est agencée dans ou sur le tuyau (10).

5. Bandage selon l'une des revendications précédentes, **caractérisé en ce que** le tuyau (10) possède une élasticité surfacique augmentée au niveau de l'emplacement pour saillie osseuse (22).

6. Bandage selon l'une des revendications précédentes, **caractérisé en ce que** l'élément de stabilisation (32) est réalisé pour une fixation réversible depuis l'extérieur autour de l'emplacement pour saillie osseuse (22), en particulier pour appliquer une précontrainte réversible de l'extérieur contre l'emplacement pour saillie osseuse (22).

7. Bandage selon l'une des revendications précédentes, **caractérisé en ce que** l'élément de stabilisation (32) est une pelote.

8. Bandage selon l'une des revendications précédentes, **caractérisé en ce que** le tuyau (10) et dépourvu de rembourrage, au moins dans un entourage de l'emplacement pour saillie osseuse (22)

9. Bandage selon l'une des revendications précédentes, **caractérisé en ce que** l'élément de stabilisation (32) comprend
- un élément porteur (3 8) et
- un rembourrage (30) de l'élément porteur (38) tourné vers le tuyau (10),
- de sorte que l'élément porteur (38) présente, dans une direction des forces actives, en particulier dans une direction longitudinale (L), une plus forte rigidité en flexion que dans une direction transversale (B) s'étendant perpendiculairement à la direction des forces actives.

10. Bandage selon l'une des revendications précédentes, **caractérisé en ce qu'**il est réalisé comme bandage (12) pour l'articulation de la main et possède une ouverture (50) pour le pouce, et l'emplacement pour saillie osseuse (22) s'applique lors de l'utilisation contre la styloïde radiale (20).

11. Bandage selon l'une des revendications 1 à 9, **caractérisé en ce qu'**il est réalisé comme bandage pour le genou, et l'emplacement pour saillie osseuse s'applique contre la patella et/ou contre les condyles.
